# EUROPEAN PATENT APPLICATION

(11) **EP 3 099 038 A1**
(43) Date of publication of application: **30.11.2016**
(21) Application number: 15201627.5
(22) Date of filing: 21.12.2015
(51) Int. Cl.: H04L 29/08

(54) **QUALITATIVE DATA COLLECTION WITH CONDITIONS-BASED QUERY FORM**

(30) Priority: 27.05.2015 US 201562166848 P
(71) Applicant: H2 Inc., 1-1002 Georg Town, Grand Cayman (KY)
(72) Inventor: DENG, Chu-Yie, 112 Taipei, Taiwan (TW); LAI, Chih-Kuang, 105 Taipei, Taiwan (TW); CHUNG, Wen-Chun, 114 Taipei, Taiwan (TW)
(74) Representative: Carangelo, Pierluigi

(57) **Abstract**

A system for providing qualitative data collection in a communication network is provided, including at least one mobile device and a server coupled to the mobile device via the communication network. Mobile device periodically collects predetermined data from mobile user of mobile device and syncs collected predetermined data to server. Server defines multiple trigger conditions and generates a query form with a set of questions for each trigger condition, wherein each question is associated with a set of predetermined answer options. Server further determines whether any of trigger conditions is matched upon receiving collected data from mobile device, and if so, sends a notification with query form corresponding to trigger condition matched to mobile device to enable mobile device to display the query form with the set of questions associated with matched trigger condition upon receiving the notification from server to perform qualitative data collection.

## Description

### CROSS REFERENCE TO RELATED APPLICATIONS

This application claims the benefit of U.S. Provisional Application No. 62/166,848, filed May 27, 2015, the entirety of which is incorporated by reference herein.

### BACKGROUND OF THE INVENTION

### Field of the Invention

The disclosure relates generally to systems and methods for providing qualitative data collection, and, more particularly, to systems and methods for providing qualitative data collection to a mobile user in a communication network.

### Description of the Related Art

For service providers, understanding issues and needs of users that use the services provided is critical to providing high quality service. The issues and needs of users may be understood by collecting specific qualitative data from users. Collecting such data from the users, however, is often expensive, inconvenient, and time consuming.

Therefore, the need exists for a system and method for automatic qualitative data collection based on the users statuses.

### BRIEF SUMMARY OF THE INVENTION

Systems and methods for providing qualitative data collection in a communication network are provided.

An embodiment of a system for providing qualitative data collection in a communication network is provided, including at least one mobile device and a server which is coupled to the mobile device via the communication network. The mobile device periodically collects predetermined data from a mobile user of the mobile device and syncs the collected predetermined data to the server. The server defines a plurality of trigger conditions and generates a query form with a set of questions for each of the trigger conditions, wherein each question is associated with a set of predetermined answer options. The server further determines whether any of the trigger conditions is matched upon receiving the collected data from the mobile device, and if so, sends a notification with the query form corresponding to the trigger condition matched to the mobile device to enable the mobile device to display the query form with the set of questions associated with the matched trigger condition upon receiving the notification from the server to perform qualitative data collection, so as to obtain responses corresponding to the set of questions and transmits the responses to the server for further operation.

In another embodiment, a method for providing qualitative data collection in a communication network for use in a system is provided, wherein the system comprises at least one mobile device and a server, and the server is coupled to the at least one mobile device via the communication network and defines a plurality of trigger conditions and generating a query form with a set of questions for each of the trigger conditions, wherein each question is associated with a set of predetermined answer options. The method comprises the steps of periodically collecting, by the mobile device, predetermined data from a mobile user of the mobile device and syncing the collected predetermined data to the server, and determining, by the server, whether any of the trigger conditions is matched upon receiving the collected data from the mobile device, and if so, sending a notification with the query form corresponding to the trigger condition matched to the mobile device to enable the mobile device to display the query form with the set of questions associated with the matched trigger condition upon receiving the notification from the server to perform qualitative data collection, so as to obtain responses corresponding to the set of questions and transmits the responses to the server for further operation.

Other aspects and features of the present invention will become apparent to those with ordinary skill in the art upon review of the following descriptions of specific embodiments of the systems for providing qualitative data collection in a communication network and related methods.

### BRIEF DESCRIPTION OF THE DRAWINGS

The invention will become more fully understood by referring to the following detailed description with reference to the accompanying drawings, wherein:
Fig. 1 is a schematic diagram illustrating an embodiment of a system for providing qualitative data collection in a communication network of the invention;
Fig. 2 is a flowchart of another embodiment of a method for providing qualitative data collection in a communication network of the invention;
Fig. 3A is a schematic diagram illustrating an embodiment of a query form with a set of questions and answer options for a particular trigger condition of the invention; and
Fig. 3B is a schematic diagram illustrating an embodiment of responses for the query form shown in Fig. 3A of the invention.

### DETAILED DESCRIPTION OF THE INVENTION

It will be readily understood that the components of the present invention, as generally described and illustrated in the figures herein, may be arranged and designed in a wide variety of different configurations. Thus, the following more detailed description of the embodiments of the systems and methods of the present invention, as represented in the figures, is not intended to limit the scope of the invention, as claimed, but is merely representative of selected embodiments of the invention.

Furthermore, the described features, structures, or characteristics may be combined in any suitable manner in one or more embodiments. One skilled in the relevant art will recognize, however, that the invention can be practiced without one or more of the specific details, or with other methods, components, etc. In other instances, well-known structures, or operations are not shown or described in detail to avoid obscuring aspects of the invention. The following description is of the best-contemplated mode of carrying out the invention. This description is made for the purpose of illustrating the general principles of the invention and should not be taken in a limiting sense.

Embodiments of the present disclosure disclose systems and methods for providing qualitative data collection in a communication network, which can trigger automatic qualitative data collection for a set of predetermined trigger conditions when any of the predetermined trigger conditions has been matched based on the user status information received from the mobile device to enabling the service provider to easily collect data from users when needed, thereby efficiently saving cost and time for qualitative data collection. According to the embodiments of systems and methods for performing qualitative data collection, service providers for providing specific services can define different conditions and rules for triggering collection of data on the server. The service provider can define different types of questions to ask to the user for each trigger condition. An application provided on the user's mobile device can periodically enter or sync quantitative data to the server and when the status of the user meets a defined trigger condition or rule, a notification will be sent to the user's mobile device. When the user opens the notification, a form will be shown in the mobile application with a set of questions the user can enter. As soon as the user completes the form, the responses are uploaded and saved to the server for further operation. In some embodiments, the responses can further be analyzed to trigger additional qualitative data collection when needed.

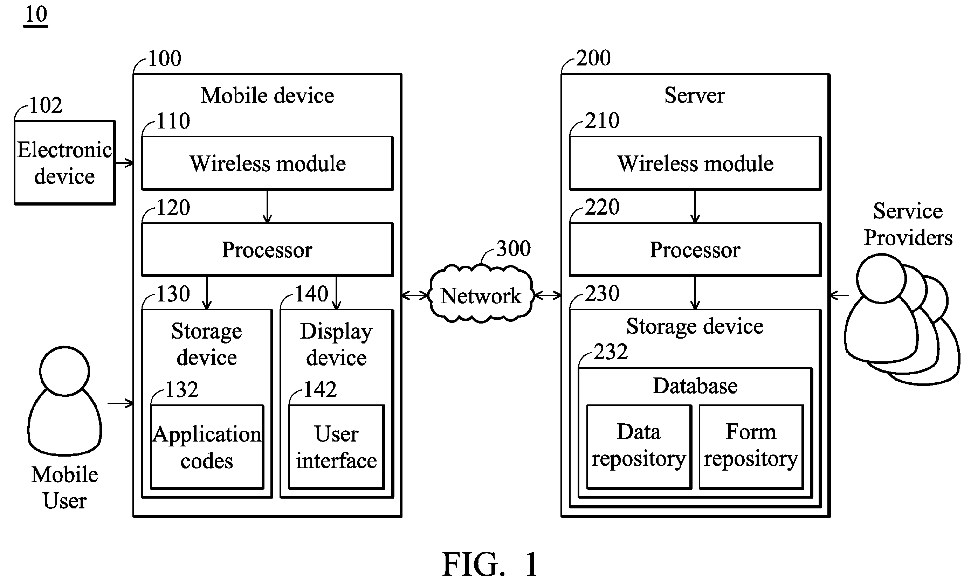

Fig. 1 is a block diagram of a system for providing quantitative data collection in accordance with an exemplary embodiment of the invention. As shown in Fig. 1, the system for providing qualitative data collection 10 may at least comprise a mobile device 100 and a server 200. The mobile device 100 and the server 200 are coupled and communicated to each other over a connected communication network 300 (e.g., any wired/wireless communication networks, such as the Internet, 3G, and/or WLAN network, etc...).

The mobile device 100 comprises a wireless module 110, a processor 120, a storage device 130 and a display device 140. The wireless module 110 receives signals from and transmits signals to a current associated network. It is to be understood that integrating the processor 120 into the wireless module 110 is also available. The wireless module 110 may be coupled to one or more antennas (not shown) and may allow communications with one or more additional devices, computers and/or servers using a wireless network. The mobile device 100 may support various communications protocols, such as the code division multiple access (CDMA), Global System for Mobile Communications (GSM), Enhanced Data GSM Environment (EDGE), High-Speed Downlink Packet Access (HSDPA), Wi-Fi (such as IEEE 802.11a/b/g/n), Bluetooth, and Wi-MAX communication protocol, and a protocol for emails, instant messaging (IM), and/or a short message services (SMS), but the invention is not limited thereto. The processor 120 may be one or more data processors, image processors and/or central processors, which are capable of executing one or more types of computer readable medium stored in the storage device 130 such as a memory. In some embodiments, the wireless module 110 may be a cellular modem that provides mobile communication functionality based on the capabilities of the underlying hardware.

The storage device 130 may be a memory of the mobile device 100 and also may be an external storage card, such as a smart media (SM) card or secure digital (SD) card, for example. The application codes 132 stored in the storage device 130 are executed by the processor 120 to activate a mobile application (hereinafter also referred to as a mobile app) to display a user interface (UI) 142 on the display device 140 (e.g. a touch panel) for the user to input data and perform the qualitative data collection. The display device 140 is configured to display related data, such as texts, figures, interfaces, and/or related information. It is understood that, in some embodiments, the display device 140 may be integrated with a touch-sensitive device (not shown). The touch-sensitive device has a touch-sensitive surface comprising sensors in at least one dimension to detect contact and movement of at least one object (input tool), such as a pen/stylus or finger near or on the touch-sensitive surface. Users can input related commands or signals via the screen of the display device 140.

The processor 120 which is coupled to the wireless module 110, the storage device 130 and the display device 140 can control the wireless module 110, the storage device 130 and the display device 140 to perform the method for providing qualitative data collection of the present invention, which will be discussed further in the following paragraphs.

To be more specific, the mobile devices 100 may be coupled to the server 200 via the connected communication network 300 (e.g., any wired/wireless communication networks, such as the Internet, 3G, and/or WLAN network, etc...).

The mobile device 100 may include or be further coupled to one or more electronic device 102 which comprises at least one or more sensors/detectors (not shown), wherein the sensors/detectors are used for collecting/measuring various sensor data, such as various health-related vital data. The electronic device 102 may perform a measurement to obtain health-related data. The electronic device 102 can be any electronic device which can collect monitored/sensor data (e.g. health-related data) via its sensor, such as medical devices and health-related monitoring devices with sensors and detectors to collect/measure medical and health indexes such as Blood Glucose level, Blood Pressure, EKG, Cholesterol, Uric Acid and so on. The medical device can be, but is not limited to, blood glucose meters, blood pressure meters, pulse oximeters, ventilators, EKG devices, thermometers, and various other vital data monitoring devices. The health-related monitoring devices can be, but is not limited to, pedometers, movement monitoring devices, sleep monitoring devices, and various other health-related monitoring devices.

The mobile device 100 may provide predetermined user information of a user of the mobile device 100 (hereinafter also referred to as a mobile user) to the server 200. For example, in one embodiment, the mobile user of the mobile device 100 can be a patient and the predetermined user information can comprise patient-related data, which may comprise personal information about the patient (such as name, gender, telephone number, date of birth, or e-mail of the patient), the patient's vital information (e.g. the measured Blood Glucose level, Blood Pressure, EKG, Cholesterol, Uric Acid and so on) and/or other health-related data, such as food intake records, exercise records, medication records and so on. The mobile device 100 may provide measured data obtained from the electronic device 102 (e.g. the medical device) or the health-related data recorded on the mobile device 100 as the user information to the server 200 each time a new measurement is performed on the user of the mobile device 100 via the electronic device 102 or a new data is recorded by the user of the mobile device 100. For example, in one embodiment, the electronic device is a blood glucose meter and the mobile device 100 (e.g. a smart phone) may provide measured data (e.g. the Blood Glucose level or Blood Pressure of the patient) obtained from the blood glucose meter as the user information to the server 200 when there is a new measurement performed on the user of the mobile device 100 and thus the server 200 can have the most recently measured data about the mobile user. In one embodiment, the mobile user may record health-related data (e.g. body weight or food intake) on the mobile device 100, the mobile device 100 may further provide the health-related data recorded by the user to the server 200 periodically (e.g., three time per day or per week) or when there is new data recorded.

The mobile device 100 may further provide a user interface 142 (e.g. an application) to obtain predetermined user data, such as the medical and health-index data (e.g. the Blood Glucose value) which may be collected or measured by the electronic devices 102 or manually inputted by the mobile user, and provide information or further operations corresponding to the monitored data. The user interface 142, such as an application which is implemented by software (a so-called App), can provide a rich, friendly, and informative user experience based on data collection and analysis. For example, the App can display/provide the following information or functionalities: enable users to manually enter health-related data, provide a logbook, provide periodic summaries during a predefined time period (e.g. 7, 14, 30 day, etc.), identify high and low trends, send real-time alerts or analytics result to users and so on.

In some embodiments, the mobile device 100 can be a PDA, a smart phone, a mobile phone, a tablet, an MID, a laptop computer, a car computer, a digital camera, a multimedia player or a game device, or any other type of mobile computational device, however, it is to be understood that the invention is not limited thereto.

The server 200 may also include a wireless module 210, a processor 220 and a storage device 230. Functionalities and structures of the wireless module 210, the processor 220 and the storage device 230 are similar to those of the wireless module 110, the processor 120 and the storage device 130. The storage device 230 further includes a database 232 which has a data repository and a form repository, wherein the data repository stores user data and qualitative data obtained from the mobile user of the mobile device 100 and the form repository stores a set of query forms corresponding to a set of predetermined trigger conditions or rules defined by the service providers. The server 200 may communication between the mobile device 100 and a service provider (e.g., a care provider or hospital's server or a doctor). The server 200 can enable the mobile device 100 and the service provider to connect and to access a platform provided by the server 200. In some embodiments, the server 200 may further provide a user interface, e.g., a specific web application, as the platform to provide a web service that allows service providers to access the data stored on the server 200 (such as user information) from a webpage corresponding to the web service so that the service providers may access the data stored on the server 200 and modify trigger conditions and the design of the query forms via the specific web application.

The processor 220 which is coupled to the wireless module 210 and the storage device 230 can control the wireless module 210 and the storage device 230 to perform the method for providing qualitative data collection of the present invention, which will be discussed further in the following paragraphs.

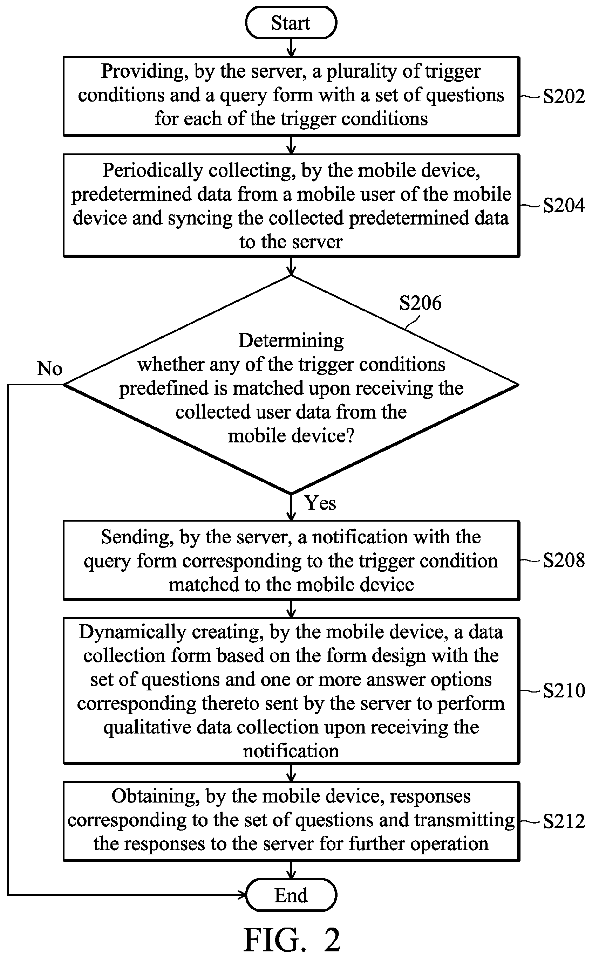

Fig. 2 is a flowchart of an embodiment of a method for providing qualitative data collection in a communication network of the invention. The method can be applied to the system 10 as shown in Fig.1 and performed by the processor 120 and the processor 220 as shown in Fig.1.

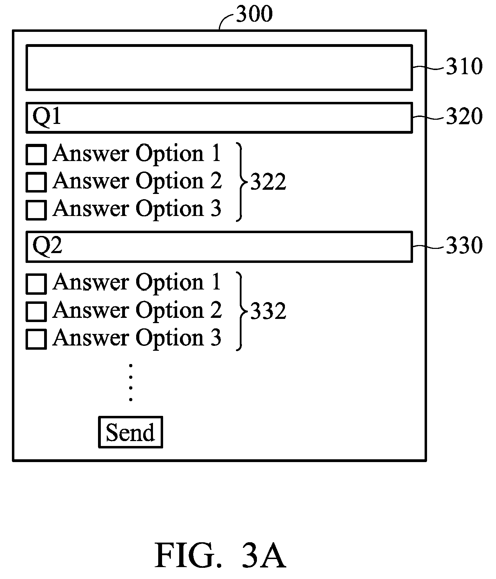

First, in step S202, the server 200 provides a plurality of trigger conditions and generates a query form with a set of questions for each of the trigger conditions, wherein each question is associated with a set of predetermined answer options. The trigger conditions and respective query forms may be defined by different service providers. To be more specific, service providers may predefine a set of different trigger conditions on the server 200 through the user interface provided by the server 200. For example, a service provider for a weight loss management service may define a trigger condition as "user gains 1 kilogram in the past 1 week". Then, the service provider also defines a query form with a set of questions corresponding to the trigger condition that service provider would like the user to answer. The service provider can define different types of questions, such as multiple choice, check box, or text response and so on. Fig. 3A is a schematic diagram illustrating an embodiment of a query form with a set of questions and answer options for a particular trigger condition of the invention. As shown in Fig. 3A, a query form 300 is illustrated, wherein the query form 300 at least includes questions 320 and 330 and the question 320 is associated with answer options 322 and the question 330 is associated with answer options 332. For example, in one embodiment, based on the trigger condition above, a service provider may define Question 1 as "How has your diet changed in the past week?" and ask user to check off the below answer options: (1) I have been eating out more often (2) My appetite has increased (3) I'm eating different types of foods. (4) None of the above. Question 2 may be defined as, "What types of foods have you been consuming more?", with the below choices or answer options: (1) Carbohydrates (2) Fats (3) Proteins (4) Fruits (5) Veggies and(6) None of the above. The service provider can further link Question 1 and Question 2 so that users who check off option 3 may be prompted with Question 2.

Then, in step S204, the mobile device 100 periodically collects predetermined data from a mobile user of the mobile device 100 and syncs the collected predetermined data to the server 200. Data collection may be achieved by a mobile app installed or activated on the mobile device 100. The mobile app may provide a user interface for routinely collecting the predetermined data from the mobile user. In some embodiments, the predetermined data may be manually inputted by the mobile user of the mobile device. In some embodiments, the predetermined data comprises health-related data. The predetermined data may be automatically obtained from an electronic device (e.g., the electronic device 102), wherein the electronic device performs a measurement to obtain the health-related data. For example, the electronic device comprises a medical device and/or a health-related monitoring device with sensors and detectors to perform the measurement to collect or measure medical and health-index data so as to generate the health-related data. The predetermined data collected will be uploaded to the server 200 whenever possible.

Upon receiving the collected user data uploaded by the mobile device 100, in step S206, the server 200 stores the uploaded user data to the database 232 of the server 200 and determines whether any of the trigger conditions predefined in the database 232 is matched. The server 200 stores the uploaded user data to the database 232 of the server 200 and routinely scans and analyzes the data in the database 232. If it is determined that none of the trigger conditions predefined in the database 232 is matched (No in step S206), the flow ends and the user data received is being stored in the database 232 of the server 200 for further operation or for subsequent analysis.

When the user's data matches a pre-defined trigger condition or rule, it is determined that one of the trigger conditions predefined in the database 232 is matched (Yes in step S206) and in step S208, the server 200 sends the notification with the query form corresponding to the trigger condition matched to the mobile device 100. To be more specific, whenever the user's data matches a pre-defined trigger condition or rule, the server 200 pulls the corresponding form of the matched trigger condition from the form repository in the database 232 and sends the form design along with a notification to the mobile user's mobile app to enable the mobile device 100 to display the query form.

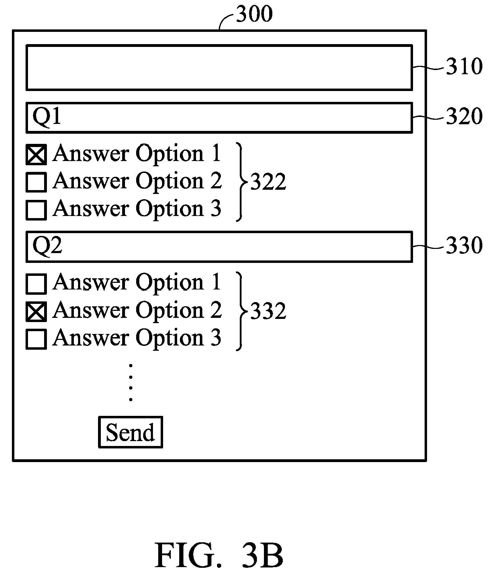

When the mobile user receives and opens the notification, in step S210, the mobile app of the mobile device 100 dynamically creates a data collection form based on the form design with the set of questions and one or more answer options corresponding thereto sent by the server 200 to perform qualitative data collection. After the qualitative data collection is completed, in step S212, the mobile device 100 obtains responses corresponding to the set of questions and transmits the responses to the server 200 for further operation. To be more specific, the mobile user may enter his/her responses using the query form. Each query form may include a set of questions corresponding to a specific trigger condition and each question may be associated with one or more of answer options related to the question. The mobile user may select one of the answer options as an answer to the respective question to enter his/her responses. Fig. 3B is a schematic diagram illustrating an embodiment of responses for the query form shown in Fig. 3A of the invention. As shown in Fig. 3B, the responses for the questions 320 and 330 are the answer options being selected in the answer options 322 and 332, respectively. The responses may then be uploaded to the server 200, and stored in the data repository in the database 232.

In some embodiments, the responses may further trigger another form to be sent to the user to trigger an additional qualitative data collection if a trigger condition is defined and matched based on the responses of the query form.

Taking blood glucose management for diabetics as an example, hypoglycemia (low blood glucose) is a serious condition that needs to be dealt with promptly and carefully. In such embodiments, the method for providing qualitative data collection of the invention can not only collect information regarding how patients responded to hypoglycemia, it can also serve to remind them of the symptoms and actions needed for it. In this embodiment, a service provider may define a first trigger condition called "Frequent pre-meal low BG" associated with a first rule of "3 or more before meal blood glucose values in the past 2 weeks that are below 70 mg/dL" on the server 200 and define a notification message that will be sent to the user corresponding to the above trigger condition as follows: "Hi there, we noticed you've been experiencing low blood sugar before your meals recently, would you like to learn more about how to deal with low blood glucose?". A respective form with a set of questions and answer options that will be sent when the user responds to the notification message is defined as follows:
Q1. "Are you feeling uncomfortable?"
A. "Yes" => Go to question 2
B. "No" => Go to question 3
Q2. "What symptoms are you feeling?"
A. "Dizziness"
B. "Feeling hungry"
C. "Shivering"
D. "Feeling listless"
E. "Cold Sweat"
Q3. "What actions have you taken to deal with low blood glucose?"
"Drank a glucose-rich drink"
"Ate something"
"Slept"
"Something else"

In operation, the mobile user may enter or sync blood glucose records to a mobile app periodically. The records are then uploaded to the server 200 by the mobile app. An example of user's blood glucose records is listed in the following Table 1:

**Table 1**

| Date | Period | Blood Glucose Value |
|---|---|---|
| 5/19/15 11:25 AM | Before lunch | 53 mg/dL |
| 5/19/15 2:13 PM | After lunch | 154 mg/dL |
| 5/20/15 11:47 AM | Before lunch | 68 mg/dL |
| 5/20/15 1:45 PM | After lunch | 136 mg/dL |
| 5/23/15 11:18 AM | Before lunch | 62 mg/dL |
| 5/23/15 2:01 PM | After lunch | 126 mg/dL |

Then, the server 200 scans the data entered by the user. Referring to the user's blood glucose records in the Table 1, as the first rule of "3 or more before meal blood glucose values in the past 2 weeks that are below 70 mg/dL" for the first trigger condition "Frequent pre-meal low BG" has been meet, the first trigger condition has been matched and thus the server 200 sends the above-mentioned notification to the mobile device 100. The mobile user of the mobile device 100 may open the notification, and the notification triggers the form designed by the service provider to be shown in the user's mobile app.

The user fills out the form by using the answer options to provide responses for the above-defined questions (e.g. selecting the answer option A or B as the answer (response) to the question Q1), and the responses to the form are sent back to the server 200. The server 200 may then scan the answers provided for the form. In this case, the answers do not further trigger another form to be sent.

In some embodiments, upon receiving the notification from the server 200 and prior to the displaying of the query form with the set of questions corresponding to the matched trigger condition, the mobile device 100 further provides an option to enable the mobile user to select whether to display the query form. If the query form is selected not to be displayed by the mobile user, the server 200 may display media data regarding solutions or suggestions corresponding to the matched trigger condition. For example, the server 200 may automatically display information, which may be, for example, in the form of video, audio, image, or text, regarding how to eat and how to solve the low BG problem for reference when the first trigger condition "Frequent pre-meal low BG" has been matched and the mobile user selects not to displaying the query form.

In some embodiments, the query form may further provide an option to include a user-provided entry not selected from the set of predetermined answer options. For example, the user-provided entry may be another answer other than the set of predetermined answer options such as a note or suggestion from the mobile user. The mobile app may transmit the responses, including with the user-provided entry, to the server 200 for analysis or display.

In another embodiment, feedback regarding an e-commerce service can also be collected. For an e-commerce service provider, customer loyalty is vital for sustaining a steady revenue stream from its users. An e-commerce service may want to find out why some of its mobile application users have discontinued buying items through their service, so the method for providing qualitative data collection of the invention can also automatically collect feedback on their behalf.

In this embodiment, the service provider for a specific e-commerce service may define a second trigger condition called "Stopped purchasing recently" with the following rule: "Purchased an item 3-6 months ago, no purchasing behavior or logged in operation in the past 0-2 months".

The service provider may define a notification message corresponding to the second trigger condition as follows: "Hi there, we would like your feedback on how we can further improve our shopping services. Could you take a moment to help us answer a few questions?" and the service provider creates a form with the below questions Q1-Q4 and a set of answer options A-F for each question as follows:
Q1. In general, how would you rate your satisfaction with our service?
A. Horrible
B. Poor
C. Average
D. Good
E. Excellent
Q2. What do you like best about our service?
A. Variety of merchandise
B. Pricing
C. Ease of use
D. Delivery speed
E. Customer service
F. Other
Q3. What areas do you think this service can be improved?
A. Variety of merchandise
B. Pricing
C. Ease of use
D. Delivery speed
E. Customer service
F. Other
Q4. Any reason why you haven't purchased anything recently?
A. I did not see anything I like
B. I am trying to cut down on my expenses
C. I have to started to use another e-commerce service
D. I don't think I'm getting the best prices here
E. I have been buying more at physical retailers
F. Other

In this embodiment, a user of this service bought a smartphone case 4 months ago and a pair of basketball shoes 3 months ago; however, this user has only browsed the app for the past couple of months without buying anything. As the server scans the user's data and determines that the user fulfills the second trigger condition "Stopped purchasing recently" and thus a notification corresponding thereto is sent to the user.

Upon receiving the notification, the user opens the notification and the notification triggers the above form to be displayed. Similarly, the user is enabled to fill out the form to enter the answers related to the particular questions by selecting one of the answer options to generate responses, and the responses to the form are sent back to the server 200. The customer service team may then store the responses and run a report to compile statistics (e.g., top answers) for people have completed this form.

In one embodiment, the predetermined data is manually inputted by the mobile user of the mobile device. In one embodiment, the predetermined data comprises health-related data. In one embodiment, the health-related data is automatically obtained from an electronic device, wherein the electronic device performs a measurement to obtain the health-related data. In one embodiment, the server further provides a user interface for enabling a service provider to configure the trigger conditions and the query form corresponding thereto.

Therefore, the systems and related methods for providing qualitative data collection in a communication network can trigger automatic qualitative data collection for a set of predetermined trigger conditions when any of the predetermined trigger conditions has been matched based on the user status information received from the mobile device to enabling the service provider to easily collect data from users when needed, thereby efficiently saving cost and time for qualitative data collection.

While the invention has been described by way of example and in terms of preferred embodiment, it should be understood that the invention is not limited thereto. Those who are skilled in this technology can still make various alterations and modifications without departing from the scope and spirit of this invention. Therefore, the scope of the present invention shall be defined and protected by the following claims and their equivalent.

## Claims

1. A system for providing qualitative data collection in a communication network (300), comprising:
at least one mobile device (100), periodically collecting predetermined data from a mobile user of the mobile device (100); and
a server (200) coupled to the mobile device (100) via the communication network (300), defining a plurality of trigger conditions and generating a query form with a set of questions for each of the trigger conditions, wherein each question is associated with a set of predetermined answer options;
wherein the mobile device (100) periodically syncs the collected predetermined data to the server (200) and the server (200) further determines whether any of the trigger conditions is matched upon receiving the collected data from the mobile device (100), and if so, sends a notification with the query form corresponding to the trigger condition matched to the mobile device (100) to enable the mobile device (100) to display the query form with the set of questions associated with the matched trigger condition upon receiving the notification from the server (200) to perform qualitative data collection, so as to obtain responses corresponding to the set of questions and transmits the responses to the server (200) for further operation.

2. The system of claim 1, wherein the server (200) further triggers an additional qualitative data collection to the mobile device (100) according to the responses.

3. The system of claim 1, wherein the mobile device (100) further activates or installs an application to provide a user interface (142) for collecting the predetermined data and performing the qualitative data collection when receiving the notification from the server (200).

4. The system of claim 1, wherein the predetermined data is manually inputted by the mobile user of the mobile device (100).

5. The system of claim 1, wherein the predetermined data comprises health-related data.

6. The system of claim 5, wherein the health-related data is automatically obtained from an electronic device (102), wherein the electronic device (102) performs a measurement to obtain the health-related data.

7. The system of claim 6, wherein the electronic device (102) comprises a medical device and/or a health-related monitoring device with sensors and detectors to perform the measurement to collect or measure medical and health-index data so as to generate the health-related data.

8. The system of claim 1, wherein upon receiving the notification from the server (200) and prior to the displaying of the query form with the set of questions corresponding to the matched trigger condition, the mobile device (100) further provides an option to enable the mobile user to select whether to display the query form and if the mobile user selects not to displaying the query form, displays media data regarding solutions or suggestions corresponding to the matched trigger condition.

9. The system of claim 1, wherein the query form further provides an option to include a user-provided entry not selected from the set of predetermined answer options.

10. The system of claim 1, wherein the server (200) further provides a user interface for enabling a service provider to configure the trigger conditions and the query form corresponding thereto.

11. A method for providing qualitative data collection in a communication network (300) for use in a system, wherein the system comprises at least one mobile device (100) and a server (200), and the server (200) is coupled to the at least one mobile device (100) mvia the communication network (300) and defines a plurality of trigger conditions and generating a query form with a set of questions for each of the trigger conditions, wherein each question is associated with a set of predetermined answer options, the method comprising:
periodically collecting (S204), by the mobile device (100), predetermined data from a mobile user of the mobile device (100) and syncing the collected predetermined data to the server (200); and
determining (S206), by the server (200), whether any of the trigger conditions is matched upon receiving the collected data from the mobile device (100), and if so, sending a notification (S208) with the query form corresponding to the trigger condition matched to the mobile device (100) to enable the mobile device (100) to display the query form with the set of questions associated with the matched trigger condition upon receiving the notification from the server to perform qualitative data collection, so as to obtain responses (S212) corresponding to the set of questions and transmits the responses to the server (200) for further operation.

12. The method of claim 11, further comprising:
triggering, by the server (200), an additional qualitative data collection to the mobile device (100) according to the responses.

13. The method of claim 11, wherein the mobile device (100) further activates or installs an application to provide a user interface (142) for collecting the predetermined data and performing the qualitative data collection when receiving the notification from the server (200).

14. The method of claim 11, wherein upon receiving the notification from the server (200) and prior to the displaying of the query form with the set of questions corresponding to the matched trigger condition, the mobile device (100) further provides an option to enable the mobile user to select whether to display the query form and if the mobile user selects not to displaying the query form, displays media data regarding solutions or suggestions corresponding to the matched trigger condition.

15. The method of claim 11, wherein the query form further provides an option to include a user-provided entry not selected from the set of predetermined answer options.
